# EUROPÄISCHE PATENTSCHRIFT

(11) **EP 2 884 958 B1**
(45) Veröffentlichungstag und Bekanntmachung des Hinweises auf die Patenterteilung: **29.03.2017**
(21) Anmeldenummer: 13741773.9
(22) Anmeldetag: 26.07.2013
(51) Int. Cl.: A61K 8/37, A61Q 13/00, A61Q 15/00, A61K 8/81

(54) **KOSMETISCHE ZUSAMMENSETZUNGEN MIT ZEITVERZÖGERTER WIRKSTOFFFREISETZUNG**
COSMETIC COMPOUNDS HAVING TIME-DELAYED ACTIVE INGREDIENT RELEASE
COMPOSITIONS COSMÉTIQUES À LIBÉRATION RETARDÉE DES ACTIFS

(30) Priorität: 17.08.2012 DE 102012214662
(43) Veröffentlichungstag der Anmeldung: 24.06.2015
(73) Patentinhaber: Henkel AG & Co. KGaA, 40589 Düsseldorf (DE)
(72) Erfinder: DÖRING, Thomas, 41540 Dormagen (DE); SCHEVARDO, Natascha, 40699 Erkrath (DE)
(86) Internationale Anmeldenummer: PCT/EP2013/065813
(87) Internationale Veröffentlichungsnummer: WO 2014/026842

(56) Entgegenhaltungen:
- EP-A1- 1 584 330
- EP-A2- 0 397 245
- EP-A2- 0 908 174
- WO-A1-02/060399
- WO-A2-2004/084844
- DE-A1- 2 731 318
- US-A- 4 152 272
- US-A- 4 731 243
- US-A1- 2005 031 565

## Beschreibung

Die vorliegende Anmeldung betrifft Partikel aus einem ausgewählten Wachsmaterial und einem Riechstoff oder Kühlwirkstoff sowie wasserhaltige kosmetische Zusammensetzungen, insbesondere Antitranspirant-Zusammensetzungen und Deodorant-Zusammensetzungen, die diese Partikel enthalten. Die Wachspartikel dienen als Retard-Träger, aus dem unter dem Einfluss der Hautwärme nach und nach die enthaltenen Riechstoffe oder Kühlwirkstoffe freigesetzt werden.

Es gibt zahlreiche Möglichkeiten, schweißhemmende Zusammensetzungen auf die Haut aufzutragen. Formstabile Stiftmassen werden aus einem Stiftspender heraus über die Haut gestrichen, bis eine wirksame Menge aufgetragen ist. Auch Gele und Cremes können mit stiftähnlichen Dispensern, die mit einer Dispenseroberfläche über die Haut gestrichen werden, aufgetragen werden. Insbesondere für schweißhemmende und/oder deodorierende Zusammensetzungen für den Achselbereich wurden zahlreiche verschiedene Applikationsformen entwickelt, neben den bereits genannten vor allem die treibgashaltigen und treibgasfreien Sprays und die Roll-on-Zusammensetzungen. Bei letzteren wird eine leicht verdickte Flüssigkeit aus einem Vorratsbehälter über eine drehbar gelagerte Kugel durch Rollen über die Haut appliziert. Schweißhemmende Rollon-Zusammensetzungen können wasserfrei und ölbasiert sein; beispielsweise ist der ölbasierte Sud üblicher Antitranspirant-Sprays auch zur Darreichung als Rollon geeignet. Hierbei liegt der schweißhemmende Wirkstoff als suspendiertes Pulver in einem Öl vor, das zur Verhinderung des Absetzens der Pulverpartikel mit einem lipophilen Geliermittel verdickt ist. Derartige Rollons sind allerdings im Markt kaum vertreten. Übliche schweißhemmende Roll-on-Zusammensetzungen sind wasserbasiert, das heißt, sie enthalten ca. 50 Gew.-% und mehr ihres Gesamtgewichts an Wasser. Der Antitranspirant-Wirkstoff, üblicherweise eine schweißhemmende Aluminium- oder Aluminium-Zirconium-Verbindung, liegt in gelöster Form vor. Eine Verdickung ist hier erforderlich, um die Applizierbarkeit der Zusammensetzung mit einem Rollapplikator zu ermöglichen.

Der Einsatz bestimmter Verdickungsmittel kann dazu führen, dass der Dufteindruck der Mittel als inhomogen wahrgenommen wird. Oft ist der Dufteindruck direkt nach der Anwendung sehr stark und wird zum Teil als aufdringlich empfunden, während einige Stunden nach der Applikation oft ein zu schwacher Dufteindruck wahrgenommen wird.

Ein weiterer Nachteil bekannter Antitranspirant-Rollons, die als Emulsion vorliegen, ist deren mangelnde Temperaturstabilität. Hier kann es, beispielsweise bei starken Temperaturschwankungen, denen die Produkte während Transport und Lagerung ausgesetzt sein können, zur Koaleszenz der Tröpfchen der dispergierten Phase kommen, was die Produkteigenschaften beeinträchtigt. Hiervon sind auch Duftstoffe betroffen.

Eine Aufgabe der vorliegenden Anmeldung war es daher, wasserbasierte Antitranspirant-Rollons mit verbesserter Langzeitstabilität, insbesondere verbesserter Langzeitduftwirkung bereitzustellen.

Eine weitere Aufgabe der vorliegenden Anmeldung war es, Antitranspirant-Rollons in Form von wasserhaltigen Emulsionen mit verbesserter Temperaturstabilität bereitzustellen.

US 4 731 243, WO 02/060399, EP 0 397 245, EP 0 908 174 und WO 2004/084844 sind auf die Bereitstellung von Parfum-Partikeln mit verbesserter Duftstabilität und Langzeitduftwirkung gerichtet. Der Gegenstand der vorliegenden Erfindung unterscheidet sich von der Lehre dieser Dokumente durch den Einsatz eines Polyalphaolefinwachses mit einem Schmelzpunkt von 40 bis 48°C sowie durch den Schmelzpunkt der Polymerpartikeln von 24 bis 34 °C.

Überraschend wurde gefunden, dass der Einsatz von Polyalphaolefinwachsen bestimmte Wirkstoffe, insbesondere Duftstoffe so konfektioniert, dass eine deutliche Verbesserung der Temperatur- und Duftstabilität und eine deutlich verbesserte Langzeitduftwirkung erzielt werden. Gegenstand der vorliegenden Anmeldung ist daher ein Partikel, enthaltend, jeweils bezogen auf das Partikelgewicht, 30 - 90 Gew.-% Polyalphaolefinwachs(e) und 10 - 70 Gew.-% eines oder mehrerer kosmetischer Wirkstoffe, ausgewählt aus Riechstoffen, Kühlwirkstoffen und Riechstoff-Kühlwirkstoff-Mischungen, wobei das mindestens eine Polyalphaolefinwachs ausgewählt ist aus Polyalphaolefinwachsen mit einem Schmelzpunkt im Bereich von 40 - 48 °C, gemessen nach ASTM D 36, und wobei das Partikel einen Schmelzpunkt im Bereich von 24 - 34 °C, gemessen nach ASTM D 36, aufweiset.

Die erfindungsgemäßen Partikel enthalten - bezogen auf ihr Gewicht - 30 bis 90 Gew.-% mindestens eines Polyalphaolefinwachses. Bevorzugte erfindungsgemäße Partikel enthalten 35 bis 85 Gew.-%, weiter bevorzugte Partikel 40 bis 80 Gew.-%, noch weiter bevorzugte Partikel 45 bis 77,5 Gew.-% und insbesondere bevorzugte Partikel 50 bis 75 Gew.-% Polyalphaolefinwachs(e). Polyalphaolefinwachse sind an sich bekannt und beispielsweise durch Polymerisation von alpha-Olefinen zugänglich. Erfindungsgemäß besonders geeignete Poly-α-Olefine lassen sich durch dehydratisierende Polymerisation von primären Alkoholen in Gegenwart saurer Aluminoschichtsilikate einer bei einer Temperatur im Bereich von 60 bis 340 °C erhalten. Vorzugsweise wird der primäre Alkohol dabei ausgewählt aus der Gruppe
a) ungesättigter monofunktioneller Alkohole,
b) verzweigter monofunktioneller Alkohole und
c) difunktioneller Alkohole.

Die Umsetzung des primären Alkohols erfolgt in diesem Verfahren bevorzugt unter Schutzgas unter kontinuierlicher Abscheidung des entstehenden Wassers. Das als Katalysator eingesetzte saure Aluminoschichtsilikat hat bevorzugt eine Säurebeladung von 3 bis 300 mval/100g. Beispiele für Aluminoschichtsilikate sind Talk sowie Tone mit Blattstruktur wie Kaolinit, Montmorillonit, Bentonite und Hectorite. Sinnvoll ist es, die Umsetzung unter Wasserabscheidung solange durchzuführen, bis keine weitere Abspaltung von Wasser mehr erfolgt. Die Reaktionszeiten liegen üblicherweise im Bereich von 2 bis 48 Stunden. Anschließend wird der Katalysator beispielsweise durch Filtration entfernt. Der Oligomerisierungsgrad der Poly-α-Olefine liegt im Bereich von 1 bis 10. Eine gezielte Einstellung des Oligomerisierungsgrades kann dadurch erreicht werden, dass man das bei der kontinuierlichen Wasserabscheidung mitgeschleppte Olefin wieder in die Reaktionsmischung zurückführt; was zu höheren Oligomerisierungsgraden führt. Die erhaltenen Poly-α-Olefine sind geruchlose, farblose oder gelbliche Produkte, die flüssig oder fest sein können. Eine genaue Strukturformel für die erhaltenen Poly-α-Olefine kann nicht angegeben werden, da die fraglichen primären Alkohole unten den dehydratisierenden Bedingungen bei der Polymerisation in verschiedenste ungesättigte Monomere isomerisiert werden, die dann miteinander polymerisieren.

Die erwähnten primären Alkohole können einzeln oder im Gemisch untereinander eingesetzt werden. Während der Alkylrest der Alkohole der Gruppe b) verzweigt ist, können die Alkylreste der primären Alkohole der Gruppen a) und c) entweder geradkettig oder verzweigt sein. Die ungesättigten Alkohole können einfach oder mehrfach ungesättigt sein und sind insbesondere olefinisch ungesättigt.

Bevorzugte kosmetische Zusammensetzungen sind solche, in denen der primäre Alkohol 6 bis 72 Kohlenstoffatome und insbesondere 6 bis 24 Kohlenstoffatome aufweist.

Als Alkohol der Gruppe a) wird bevorzugt ein linearer Alkohol eingesetzt. Beispiele ungesättigter monofunktioneller Alkohole der Gruppe a) sind 10-Undecen-1-ol, Oleylalkohol, Elaidylalkohol, Ricinolalkohol, Linoleylalkohol, Linolenylalkohol, Gadoleylalkohol, Erucaalkohol und Brassidylalkohol.

Als Alkohol der Gruppe b) wird vorzugsweise ein Alkohol eingesetzt, der ausgewählt ist aus der Gruppe der verzweigten Alkohole mit b1) mindestens einer Methylgruppe und insbesondere 1 bis 6 Methylverzweigungen im Alkylrest, b2) einer C₂-C₁₈-Verzweigung im Alkylrest und b3) einer C₂-C₁₈-Verzweigung in α-Stellung zur endständigen CH₂OH-Gruppe. Im Fall der Gruppe b1) mit mindestens einer Methylverzweigung in der Alkylgruppe kann der Methylrest an jeder beliebigen Stelle in der Alkylkette positioniert sein. Geeignete Beispiele sind Isooctylalkohol, Isononylalkohol, Isostearylalkohol oder Isotridecylalkohol. Unter diesen ist Isononylalkohol besonders bevorzugt. Bei mehreren Methylgruppen beträgt deren Anzahl bevorzugt 2 bis 6, beliebig über den Alkylrest des Alkohols verteilt. Sofern es sich um Alkohole der Gruppe b2) mit einer C₂-C₁₈-Alkylgruppe als Verzweigung handelt, sind vorzugsweise keine weiteren Verzweigungen im Alkylrest des Alkohols vorhanden.

Weitere geeignete primäre monofunktionelle verzweigte Alkohole sind die dem Fachmann bekannten Guerbetalkohole, die bekanntlich durch Dimerisierung von Fettalkoholen zugänglich sind und sich strukturell dadurch auszeichnen, dass sie in α-Stellung zur endständigen CH₂OH-Gruppe einen längeren Alkylrest, vorzugsweise mit 2 bis 18 Kohlenstoffatomen, aufweisen. Geeignete Guerbetalkohole sind 2-Hexyldecanol, 2-Butyloctanol, 2-Octyldodecanol und 2-Hexyldecylpalmitat/stearat, 2-Ethylhexanol und 2-Propylheptanol. Bevorzugt ist 2-Ethylhexylalkohol.

Als Alkohole der Gruppe c), also difunktionelle Alkohole (mit 2 Hydroxylgruppen), können gesättigte oder ungesättigte Diole eingesetzt werden wie 1,5-Pentandiol, 1,8-Octandiol, 1,6-Hexandiol, 1,10-Decandiol, 1,12-Dodecandiol, 1,12-Octadecandiol oder die dem Fachmann bekannten Dimerdiole.

Die Poly-α-Olefine können in den erfindungsgemäßen Partikeln in ungesättigter Form eingesetzt werden. Wegen der besseren Oxidationsbeständigkeit ist es jedoch bevorzugt, die Poly-α-Olefine im Anschluss an die dehydratisierende Polymerisation zu hydrieren und sie in der hydrierten (gehärteten) Form in den erfindungsgemäßen Partikeln zu verwenden.

Die kann dabei in an sich bekannter Weise bei Temperaturen im Bereich von 150° bis 250°C, vorzugsweise 190° bis 210 °C, und Drücken von 20 bis 150 bar (Niederdruck-Verfahren) oder 150 bis 350 bar (Hochdruck-Verfahren) erfolgen. Als Katalysatoren eignen sich die aus dem Stand der Technik bekannten Hydrlerkatalysatoren wie Nickel oder die Edelmelallkatalysatoren, insbesondere auf Basis von Platin oder Palladium. Als besonders geeignet Edelmatallkatalysatoren haben sich Palladiumkatalysatoren erwiesen, insbesondere Palladium auf Kohle. Man kann den Katalysator In Form einer Suspension oder in fester Form zu den Poly-α-Olefinen in üblichen Mengen geben, die für das bevorzugte Palladium auf Kohle Im Bereich von 0,001 bis 5 Gew.% - berechnet als Palladium - liegt. Es ist jedoch auch möglich, den Katalysator auf einem festen Trägermaterial anzuordnen wie Aktivkohle, Graphit, Kieselgur, Kieselgel, Spinelle, Aluminiumoxid oder keramischen Materialien. Ebenso haben sich die Nickelkatalysatoren, beispielsweise suspendiertes Nickel wie Nysofact 101 I a (Fa. Engelhard) als geeignet erwiesen, welches vorzugsweise in Mengen von 0,01 bis 5 Gew.% - bezogen auf Nickel - eingesetzt wird.

Die beschriebenen Poly-α-Olefine sind, wie erwähnt, farblose bis leicht gelbliche, praktisch geruchlose Verbindungen mit hohen Spreitwerten, typischerweise größer als 1000 mm²/10 Minuten und vorzugsweise größer als 1600 mm²/10 Minuten (Definition nach Zeidler) liegen. Wenn nachfolgend allgemein von Poly-α-Olefinen die Rede ist, sind damit sowohl die hydrierten als auch die nicht hydrierten Verbindungen gemeint.

Über die Auswahl der Poly-alpha-Olefine lässt sich das Eigenschaftsprofil der erfindungsgemäßen Partikel variieren. Hier sind erfindungsgemäße Partikel bevorzugt, bei denen das mindestens eine Polyalphaolefinwachs ausgewählt ist aus Polyalphaolefinwachsen mit einem Schmelzpunkt im Bereich von 30 - 75 °C, bevorzugt 35 - 60°C, besonders bevorzugt 40 - 55°C, außerordentlich bevorzugt 40 - 48 °C, gemessen nach ASTM D 36.

Die erfindungsgemäßen Partikel enthalten - bezogen auf ihr Gewicht - 10 - 70 Gew.-% eines oder mehrerer kosmetischer Wirkstoffe, ausgewählt aus Riechstoffen, Kühlwirkstoffen und Riechstoff-Kühlwirkstoff-Mischungen.

Beispiele für Duft- und Riechstoffverbindungen vom Typ der Ester sind Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Beispiele für Duft- und Riechstoffverbindungen vom Typ der Ether sind Benzylethylether und Ambroxan, Beispiele für Duft- und Riechstoffverbindungen vom Typ der Aldehyde sind die linearen Alkanale mit 8 - 18 C-Atomen, Citral, Citronellal, Citronellyloxy-acetaldehyd, Cyclamenaldehyd, Lilial und Bourgeonal, Beispiele für Duft- und Riechstoffverbindungen vom Typ der Ketone sind die Jonone, alpha-Isomethylionon und Methylcedrylketon, Beispiele für Duft- und Riechstoffverbindungen vom Typ der Alkohole sind Anethol, Citronellol, Eugenol, Geraniol, Linalool, Phenylethylalkohol und Terpineol, Beispiele für Duft- und Riechstoffverbindungen vom Typ der Terpene sind Limonen und Pinen. Beispiele für Duft- und Riechstoffverbindungen sind Pine-, Citrus-, Jasmin-, Patchouly-, Rosen-, Ylang-Ylang-Öl, Muskateller-Salbeiöl, Kamillenöl, Nelkenöl, Minzöl, Zimtblätteröl, Lindenblütenöl, Wacholderbeeröl, Vetiveröl, Olibanumöl, Galbanumöl, Labdanumöl, Orangenblütenöl, Neroliöl, Orangenschalenöl und Sandelholzöl, weiterhin die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Niaouliöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Wacholderbeeröl, Wermutöl, Wintergrünöl, Ysop-Öl, Zimtöl, Zitronellöl, Zitronenöl und Zypressenöl. Weitere Duft- und Riechstoffverbindungen sind Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Iso-eugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadecanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, gamma-Undecalacton, Vanillin, Veratrumaldehyd, Zimtaldehyd, Zimatalkohol, Zimtsäure, Zimtsäureethylester und Zimtsäurebenzylester. Weitere (leichter flüchtige) Riechstoffe sind Alkylisothiocyanate (Alkylsenföle), Butandion, Limonen, Linalool, Linaylacetat und -propionat, Methyl-n-heptenon, Phellandren, Phenylacetaldehyd, Terpinylacetat, Zitral und Zitronellal.

Bevorzugt werden Mischungen verschiedener Duftstoffe verwendet, die gemeinsam eine ansprechende Duftnote erzeugen.

Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl.

Als Kühlwirkstoff sind erfindungsgemäß solche Verbindungen anzusehen, die, ähnlich wie I-Menthol, die Thermorezeptoren in der Haut und den Schleimhäuten so stimulieren, dass ein kühler sensorischer Eindruck entsteht. Insbesondere der Rezeptor CMR-1 ("cold- and menthol-sensitive receptor"), der zur Familie der TRP-Kanäle gehört, wird durch die Kühlwirkstoffe stimuliert, wodurch ein Kälteeindruck erzeugt wird.

Erfindungsgemäß geeignete Kühlwirkstoffe sind ausgewählt aus 2-lsopropyl-N,2,3-trimethylbutyramid (FEMA 3804), N-Ethyl-p-menthane-3-carboxamid (FEMA 3455), insbesondere 1 R,3R,4S- N-Ethyl-p-menthane-3-carboxamid, Ethyl-3-(p-menthan-3-carboxamido)acetat (FEMA 4309), (1R,2S,5R)-N-(4-Methoxyphenyl)-p-menthancarboxamid (FEMA 4681), N-Ethyl-2,2-diisopropylbutanamid (FEMA 4557), N-Cyclopropyl-5-methyl-2-isopropylcyclohexancarboncarboxamid (FEMA 4693), N-(4-cyanomethylphenyl)-p-menthancarboxamid (FEMA 4496), N-(2-(Pyridin-2-yl)ethyl)-3-p-menthancarboxamid (FEMA 4549), N-(2-Hydroxyethyl)-2-isopropyl-2,3-dimethylbutanamid (FEMA 4602), N-(1,1-Dimethyl-2-hydroxyethyl)-2,2-diethylbutanamid (FEMA 4603), (2S,5R)-N-[4-(2-Amino-2-oxoethyl)phenyl]-p-menthancarboxamid (FEMA 4684), 2-[(2-p-Menthoxy)ethoxy]ethanol (FEMA 4718), (2,6-Diethyl-5-isopropyl-2-methyltetrahydropyran (FEMA 4680), 3-(I-Menthoxy)-2-methylpropan-1,2-diol (FEMA 3849), p-Menthan-3,8-diol, insbesondere (+)-cis-p-Menthan-3,8-diol und (-)-trans-p-Menthan-3,8-diol sowie Mischungen aus (+)-cis-p-Menthan-3,8-diol und (-)-trans-p-Menthan-3,8-diol, insbesondere eine Mischung im Gewichtsverhältnis 62:38 (FEMA 4053), (1R,3R,4S)-3-Menthyl-3,6-dioxaheptanoat, (1 R,2S,5R)-3-Menthylmethoxyacetat, (1 R,2S,5R)-3-Menthyl-3,6,9-trioxadecanoat, (1 R,2S,5R)-3-Menthyl-3,6,9-trioxadecanoat, (1 R,2S,5R)-3-Menthyl-(2-hydroxyethoxy)acetat, (1R,2S,5R)-Menthyl-11-hydroxy-3,6,9-trioxaundecanoat, (-)-Cubebol (FEMA 4497), N-(4-Cyanomethylphenyl)-p-menthancarboxamid, N,N-Dimethylmenthylsuccinamid (2-Isopropyl-5-methylcyclohexyl-4-(dimethylamino)-4-oxobutanoat, FEMA 4230), 6-Isopropyl-3,9-dimethyl-1,4-dioxaspiro[4.5]decan-2-on (FEMA 4285), N-Benzo[1,3]-dioxol-5-yl-3-p-menthancarboxamid, N-Benzoxazol-4-yl-3-p-menthancarboxamid, N-4-([1,2,4]-Triazol-l-yl)-phenyl-3-p-menthancarboxamid, N-4-(Pyrazol-l-yl)-phenyl-3-p-menthancarboxamid, N-(1-Isopropyl-1,2-dimethylpropyl)-1,3-benzodioxol-5-carboxamid, Mischungen von 2,2,5,6,6-Pentamethyl-2,3,6,6a-tetrahydropentalen-3a(1H)-ol und 5-(2-Hydroxy-2-methylpropyl)-3,4,4-trimethylcyclopent-2-en-1-on gemäß US 20070274928 A1, (1S,2S,5R)-N-(4-(Cyanomethyl)phenyl)-2-isopropyl-5-methylcyclo-hexancarboxamid, Neo-Menthyllactat (2S), Neo-Menthyllactatacetat [(1S,2S,5R)-2-Isopropyl-5-methylcyclohexyl-2(S)-acetoxypropanoat] und 1-Isopropyl-4-methyl-bicyclo[2.2.21oct-5-ene-2,3-dicarbinol gemäß WO 2007/022651 sowie Mischungen hiervon. Sofern die vorgenannten Verbindungen hinsichtlich ihrer Stereoisomere nicht näher bezeichnet sind, sind insbesondere die All-äquitorial-Isomere der vorgenannten Verbindungen als bevorzugt anzusehen.

Als Kühlwirkstoffe bevorzugt sind Menthol, Isopulegol sowie Mentholderivate, z. B. Menthyllactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Besonders bevorzugt sind Menthol, Isopulegol, Menthyllactat, Menthoxypropandiol und Menthylpyrrolidoncarbonsäure.

Bevorzugte erfindungsgemäße Zusammensetzungen enthalten einen oder mehrere Kühlwirkstoffe in einer Gesamtmenge von 0,01 - 5 Gew.-%, bevorzugt in einer Gesamtmenge von 0,05 - 2 Gew.-%, besonders bevorzugt in einer Gesamtmenge von 0,1 - 1 Gew.-%, außerordentlich bevorzugt in einer Gesamtmenge von 0,2 - 0,6 Gew.-%, wobei sich die Mengenangaben auf das Gewicht der Zusammensetzung beziehen.

Bevorzugte erfindungsgemäße Partikel enthalten 15 bis 65 Gew.-%, weiter bevorzugte Partikel 20 bis 60 Gew.-%, noch weiter bevorzugte Partikel 22,5 bis 55 Gew.-% und insbesondere bevorzugte Partikel 25 bis 50 Gew.-% eines oder mehrerer kosmetischer Wirkstoffe, ausgewählt aus Riechstoffen, Kühlwirkstoffen und Riechstoff-Kühlwirkstoff-Mischungen.

Zusammenfassend sind erfindungsgemäß bevorzugte Partikel dadurch gekennzeichnet, dass der Gesamtanteil an Polyalphaolefinwachs(en) 50 - 75 Gew.-% und der Gesamtanteil an mindestens einem kosmetischen Wirkstoff, ausgewählt aus Riechstoffen, Kühlwirkstoffen und Riechstoff-Kühlwirkstoff-Mischungen, 25 - 50 Gew.-%, beträgt, jeweils bezogen auf das Partikelgewicht.

Auch die erfindungsgemäßen Partikel (d.h. Polymer plus Wirkstoff) weisen vorzugsweise bestimmte physikalische Eigenschaften auf, die sich wiederum über die Art des Polymers und des Wirkstoffes sowie über deren Mengenverhältnis zueinander steuern lassen. Erfindungsgemäß bevorzugte Partikel sind gekennzeichnet durch einen Schmelzpunkt im Bereich von 25 - 40 °C, bevorzugt 26 - 35 °C, besonders bevorzugt 27 - 33°C, außerordentlich bevorzugt 29 - 31 °C, gemessen nach ASTM D 36.

Um im Hinblick auf die spätere Applikationsform in Kosmetika optimale Ergebnisse zu erzielen, hat es sich als besonders geeignet erwiesen, wenn die erfindungsgemäßen Partikel einen zahlenmittleren Partikeldurchmesser im Bereich von 50 nm bis 100 µm, bevorzugt 100 nm bis 80 µm, besonders bevorzugt 500 nm bis 50 µm, außerordentlich bevorzugt 1 µm bis 30 µm aufweisen. Die erfindungsgemäßen Partikel eignen sich insbesondere zum Einsatz in kosmetischen Zusammensetzungen. Besonders bewährt haben sie sich in Deodorant- und Antitranspirant-Zusammensetzungen. Bei diesem speziellen Anwendungsgebiet hat sich insbesondere die Darreichungsform des Roll-ons als besonders geeignetes Einsatzgebiet für die erfindungsgemäßen Partikel erwiesen.

Ein weiterer Gegenstand der vorliegenden Erfindung ist eine kosmetische Zusammensetzung, enthaltend Wasser und 0,01 - 10 Gew.-%, bezogen auf das Gewicht der kosmetischen Zusammensetzung, eines oder mehrerer erfindungsgemäßer Partikel.

Die erfindungsgemäßen Zusammensetzungen enthalten bevorzugt 40 - 90 Gew.-%, besonders bevorzugt 50 - 85 Gew.-%, außerordentlich bevorzugt 60 - 80 Gew.-%, weiter außerordentlich bevorzugt 65 - 75 Gew.-% Wasser, jeweils bezogen auf das Gesamtgewicht der Zusammensetzung. Mit "Wasser" ist im Sinne der vorliegenden Anmeldung "freies Wasser" gemeint, also Wasser, das nicht in Form von Kristallwasser, Hydratationswasser oder ähnlich molekular gebundenem Wasser in der Antitranspirant-Zusammensetzung enthalten ist. Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar. Freies Wasser ist solches Wasser, das beispielsweise als Lösemittel oder als Lösemittelbestandteil anderer Wirkstoffe in der erfindungsgemäßen Zusammensetzung enthalten ist.

Wie bereits erwähnt, sind bevorzugte erfindungsgemäße Zusammensetzungen Deodorantien und/oder Antitranspirantien. Erfindungsgemäß bevorzugte kosmetische Zusammensetzungen sind dadurch gekennzeichnet, dass mindestens ein Antitranspirant-Wirkstoff enthalten ist.

Erfindungsgemäß bevorzugte Antitranspirant-Wirkstoffe sind ausgewählt aus den wasserlöslichen adstringierenden anorganischen und organischen Salzen des Aluminiums, Zirkoniums und Zinks bzw. beliebigen Mischungen dieser Salze. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind. Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zinkund Natriumsalzen, den Komplexen von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat, Zirconyloxyhalogeniden, insbesondere Zirconyloxychloriden, Zirconylhydroxyhalogeniden, insbesondere Zirconylhydroxychloriden (Zirkoniumchlorohydrat). Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Aktivierte Aluminium- und Aluminium-Zirconiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminium- und Aluminium-Zirconiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminium- oder Aluminium-Zirconiumsalzes, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18-45 Gew.-% eines aktivierten Aluminium- oder Aluminium-Zirconiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminium- oder Aluminium-Zirconiumsalze mit einem mehrwertigen Alkohol, die 20 - 50 Gew.-%, besonders bevorzugt 20 - 42 Gew.-%, aktiviertes schweißhemmendes Aluminium- oder Aluminium-Zirconiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole.

Weitere bevorzugte schweißhemmende Wirkstoffe sind basische Calcium-Aluminiumsalze. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt. Weitere bevorzugte schweißhemmende Wirkstoffe sind Aluminium-Zirconium-Komplexe, die mit Salzen von Aminosäuren, insbesondere mit Alkali- und Erdalkaliglycinaten, gepuffert sind.

Weitere bevorzugte schweißhemmende Wirkstoffe sind aktivierte Aluminium- oder Aluminium-Zirconiumsalze, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(Al+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt. Weitere besonders bevorzugte feste schweißhemmende aktivierte Salzzusammensetzungen, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt. Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminium- oder Aluminium-Zirconiumsalze, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Strontiumsalz in einer solchen Menge, um ein Sr:(Al+Zr)-Gewichtsverhältnis von 1:1 - 1:28 und bevorzugt 1:2 - 1:25 bereitzustellen. Besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Hydroxyalkansäure, dass das Hydroxyalkansäure zu (AI+Zr) - Gewichtsverhältnis 2:1 - 1:20, bevorzugt 1:1 - 1:10, beträgt. Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein Al:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein Al:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen. Erfindungsgemäß bevorzugte Zirconiumsalze haben die allgemeine Formel ZrO(OH)₂₋ₐClₐ · x H₂O mit a = 1.5 - 1.87; x = 1 - 7, wobei a und x rationale Zahlen sind.

Die schweißhemmenden Wirkstoffe können sowohl in solubilisierter als auch in ungelöster, suspendierter Form vorliegen. Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen. Bevorzugte Aluminiumsalze und Aluminiumzirconiumsalze weisen ein molares Metall-zu-Chlorid-Verhältnis von 0,9 - 1,3, bevorzugt 0,9 - 1,1, besonders bevorzugt 0,9 - 1,0, auf.

Bevorzugte Aluminiumzirconiumchlorohydrate haben im allgemeinen die empirische Formel AlₙZr(OH)_{[3n+4-m(n+1)]}(Cl)_{[m(n+1)]} mit n = 2,0 - 10,0, bevorzugt 3,0 - 8,.0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:Cl = 1,1 - 0,9), und besonders bevorzugt m = 1,00 - 1,11 (entsprechend M:Cl = 1,0 - 0,9), weiterhin sehr bevorzugt m = 1,02 - 1,11 (entsprechend M:Cl = 0,98 - 0,9) sowie sehr bevorzugt m = 1,04 - 1,11 (entsprechend M:Cl = 0,96 - 0,9).

Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten. Bevorzugte schweißhemmende Salze sind Aluminium-Zirconiumtetrachlorohydrat (Al:Zr = 2-6; M:Cl = 0.9-1.3), insbesondere Salze mit einem molaren Metall-zu-Chlorid-Verhältnis von 0,9 - 1,1, bevorzugt 0,9 - 1,0.

Weiterhin erfindungsgemäß bevorzugt sind Aluminiumzirconiumchlorohydrat-Glycin-Salze, die mit Betain ((CH₃)₃N⁺-CH₂-COO⁻) stabilisiert sind. Besonders bevorzugte entsprechende Verbindungen weisen ein molares Gesamt-(Betain + Glycin)/Zr-Verhältnis von (0,1 - 3,0): 1, bevorzugt (0,7 - 1,5) : 1 und ein molares Verhältnis von Betain zu Glycin von mindestens 0,001 : 1 auf. In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet). Bevorzugte Aluminiumzirconiumsalze sind solche mit einem hohen HPLC-Peak 5-Aluminium-Gehalt (auch als "E⁵AZCH" bezeichnet). Weiterhin sind solche aktivierten "E⁵AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt. Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind.

Weitere bevorzugte Antitranspirant-Wirkstoffe sind ausgewählt aus adstringierenden Titansalzen. Die Antitranspirant-Wirkstoffe können als nicht-wässrige Lösungen oder als glycolische Solubilisate eingesetzt werden.

Besonders bevorzugte erfindungsgemäße Zusammensetzungen sind dadurch gekennzeichnet, dass der mindestens eine Antitranspirant-Wirkstoff in einer Menge von 3 - 27 Gew.-%, bevorzugt 5 - 22 Gew.-% und besonders bevorzugt 10 - 20 Gew.-%, enthalten ist, bezogen auf das Gesamtgewicht der kristallwasserfreien Aktivsubstanz (USP) in der Gesamtzusammensetzung. Erfindungsgemäß ebenfalls bevorzugte kosmetische Zusammensetzungen sind dadurch gekennzeichnet, dass mindestens ein Deodorant-Wirkstoff enthalten ist.

Erfindungsgemäß bevorzugte Deodorant-Wirkstoffe sind Geruchsabsorber, desodorierend wirkende Ionenaustauscher, keimhemmende Mittel, präbiotisch wirksame Komponenten sowie Enzyminhibitoren oder, besonders bevorzugt, Kombinationen der genannten Wirkstoffe. Silicate dienen als Geruchsabsorber, die auch gleichzeitig die rheologischen Eigenschaften der erfindungsgemäßen Zusammensetzung vorteilhaft unterstützen. Zu den erfindungsgemäß besonders bevorzugten Silicaten zählen vor allem Schichtsilicate und unter diesen insbesondere Montmorillonit, Kaolinit, Illit, Beidellit, Nontronit, Saponit, Hectorit, Bentonit, Smectit und Talkum. Weitere bevorzugte Geruchsabsorber sind beispielsweise Zeolithe, Zinkricinoleat, Cyclodextrine, bestimmte Metalloxide, wie z. B. Aluminiumoxid, sowie Chlorophyll. Sie werden bevorzugt in einer Menge von 0,1 - 10 Gew.-%, besonders bevorzugt 0,5 - 7 Gew.-% und außerordentlich bevorzugt 1 - 5 Gew.-%, jeweils bezogen auf die Gesamtzusammensetzung, eingesetzt.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzethoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Deodorant-Wirkstoffe sind ausgewählt aus so genannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier z.B. Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen, mit einbezogen.

Weitere bevorzugte Deodorant-Wirkstoffe sind ausgewählt aus den keimhemmend wirkenden Parfümölen und den Deosafe^{®}-Parfümölen, die von der Firma Symrise, vormals Haarmann und Reimer, erhältlich sind.

Zu den Enzyminhibitoren gehören Stoffe, die die für die Schweißzersetzung verantwortlichen Enzyme, insbesondere die Arylsulfatase, β-Glucuronidase, Aminoacylase, Esterasen, Lipasen und/oder Lipoxigenase hemmen, z. B. Trialkylcitronensäureester, insbesondere Triethylcitrat oder Zinkglycinat.

Bevorzugte erfindungsgemäße Deodorant- oder Antitranspirant-Zusammensetzungen sind dadurch gekennzeichnet, dass der mindestens eine Deodorant-Wirkstoff ausgewählt ist aus Arylsulfatase-Inhibitoren, β-Glucuronidase-Inhibitoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂-Alkylrest, insbesondere α-(2-Ethylhexyl)glycerinether, Phenoxyethanol, keimhemmend wirkenden Parfümölen, Deosafe^{®}-Parfümölen (Deosafe^{®} ist ein eingetragenes Warenzeichen der Firma Symrise, vormals Haarmann & Reimer), präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Lantibiotika, sowie Mischungen der vorgenannten Substanzen.

Weitere bevorzugte erfindungsgemäße Deodorant- oder Antitranspirant-Zusammensetzungen sind dadurch gekennzeichnet, dass der mindestens eine Deodorant-Wirkstoff in einer Gesamtmenge von 0,1 - 10 Gew.-%, bevorzugt 0,2 - 7 Gew.-%, besonders bevorzugt 0,3 - 5 Gew.-% und außerordentlich bevorzugt 0,4 - 1,0 Gew.-%, jeweils bezogen auf das Gesamtgewicht der Aktivsubstanz des Deodorant-Wirkstoffs oder der Deodorant-Wirkstoffe in der Gesamtzusammensetzung, enthalten ist.

Die erfindungsgemäßen Zusammensetzungen können in einer weiteren besonders bevorzugten Ausführungsform sowohl mindestens einen Deodorant- als auch mindestens einen Antitranspirant-Wirkstoff enthalten.

Die erfindungsgemäßen Zusammensetzungen können weiter mindestens eine Polymer enthalten, das ausgewählt ist aus Polysacchariden, deren Estern und/oder Ethern sowie Mischungen davon. Polysaccharide (auch als Mehrfachzucker, Vielfachzucker, Glycane/Glykane oder Polyosen bezeichnet) sind Kohlenhydrate, die aus einer großen Anzahl (mindestens 10) Monosacchariden (Einfachzuckern) über eine glycosidische Bindung verbunden sind. Es handelt sich um Biopolymere, bei denen unbekannte Anzahl Monosaccharideinheiten oder eine statistische Molekülgrößenverteilung vorliegt. Polysaccharide sind zum Beispiel Glycogen, Stärke (Amylose und Amylopektin), Pektine, Chitin, Callose und Cellulose. Ein wichtiges Heteropolysaccharid ist beispieelsweise Xanthan. Erfindungsgemäß bevorzugte Zusammensetzungen sind dadurch gekennzeichnet, daß sie das mindestens eine Polymer, das ausgewählt ist aus Polysacchariden, deren Estern und/oder Ethern sowie Mischungen hiervon, in einer Gesamtmenge von 0,05 - 1 Gew.-%, bevorzugt 0,1 - 0,7 Gew.-%, besonders bevorzugt 0,1 - 0,3 Gew.-%, jeweils bezogen auf das Gesamtgewicht der Zusammensetzung, enthalten.

Unter den Polysacchariden, deren Estern und/oder Ethern sowie Mischungen davon sind Cellulose und die entsprechenden Derivate besonders bevorzugt.

Bevorzugte Celluloseether sind ausgewählt aus Hydroxyalkylcellulosen, insbesondere aus Hydroxyethylcellulose, Hydroxypropylcellulose, Hydroxypropylmethylcellulose, Carboxymethylcellulose, Cetylhydroxyethylcellulose, Hydroxybutylmethylcellulose und Methylhydroxyethylcellulose sowie Mischungen hiervon. Außerordentlich bevorzugt ist Hydroxyethylcellulose.

Bevorzugte erfindungsgemäße Zusammensetzungen enthalten mindestens ein nichtionisches verdickendes Polymer, das ausgewählt ist aus Cellulose und Celluloseethern sowie Mischungen hiervon, in einer Gesamtmenge von 0,05 - 1 Gew.-%, bevorzugt 0,1 - 0,7 Gew.-%, besonders bevorzugt 0,1 - 0,3 Gew.-%, jeweils bezogen auf das Gesamtgewicht der Zusammensetzung. Weitere bevorzugte erfindungsgemäße Zusammensetzungen enthalten 0,05 - 1 Gew.-%, bevorzugt 0,1 - 0,7 Gew.-%, besonders bevorzugt 0,1 - 0,3 Gew.-%, Hydroxyethylcellulose, jeweils bezogen auf das Gesamtgewicht der Zusammensetzung.

Weitere bevorzugte erfindungsgemäße Zusammensetzungen enthalten, jeweils bezogen auf das Gesamtgewicht der Zusammensetzung, 0,05 - 1,0 Gew.-%, bevorzugt 0,1 - 0,8 Gew.-%, besonders bevorzugt 0,2 - 0,5 Gew.-%, Dehydroxanthan Gum und 0,05 - 1 Gew.-%, bevorzugt 0,1 - 0,7 Gew.-%, besonders bevorzugt 0,1 - 0,3 Gew.-%, Hydroxyethylcellulose.

Es hat sich gezeigt, daß die erfindungsgemäßen Zusammensetzungen mit besonderem Vorzug zusätzlich Dehydroxanthan Gum enthalten können. Mit der Kombination von Dehydroxanthan Gum mit mindestens einem nichtionischen verdickenden Polymer, das aus Cellulose und Celluloseethern sowie Mischungen hiervon ausgewählt ist, liegen die Viskositäten der erfindungsgemäßen Zusammensetzungen im anwendungstechnisch geforderten Bereich zwischen 1500 und 2500 mPas, wobei die Viskosität bei 23°C mit einem Rotationsviskosimeter der Firma Brookfield, Gerät RVF, Spindel 4, Scherrate (Umdrehungsfrequenz) 20 min⁻¹, ohne Helipath, gemessen wird.

Die erfindungsgemäßen Zusammensetzungen enthalten Dehydroxanthan Gum bevorzugt in einer Menge von 0,05 - 1 Gew.-%, besonders bevorzugt 0,1 - 0,8 Gew.-%, außerordentlich bevorzugt 0,2 - 0,5 Gew.-%, jeweils bezogen auf das Gesamtgewicht der Zusammensetzung.

Weitere bevorzugte erfindungsgemäße Zusammensetzungen sind dadurch gekennzeichnet, dass Dehydroxanthan Gum und die Gesamtmenge an nichtionischem verdickendem Polymer, ausgewählt aus Cellulose und Celluloseethern sowie Mischungen hiervon, in einem Gewichtsverhältnis von 1 bis 2,5, bevorzugt 1,2 bis 2,0, besonders bevorzugt 1,4 bis 1,6, enthalten sind.

Weitere bevorzugte erfindungsgemäße Zusammensetzungen sind dadurch gekennzeichnet, dass Dehydroxanthan Gum und Hydroxyethylcellulose in einem Gewichtsverhältnis von 1 bis 2,5, bevorzugt 1,2 bis 2,0, besonders bevorzugt 1,4 bis 1,6, enthalten sind.

Besonders bevorzugte erfindungsgemäße Zusammensetzungen sind dadurch gekennzeichnet, dass sie, jeweils bezogen auf das Gesamtgewicht der Zusammensetzung, 0,1 - 0,8 Gew.-% Dehydroxanthan Gum und 0,1 bis 0,7 Gew.-% Hydroxyethylcellulose enthalten.

Bevorzugte erfindungsgemäße Zusammensetzungen enthalten mindestens einen Öl-in-WasserEmulgator mit einem HLB-Wert größer 7 bis 20, der besonders bevorzugt aus nichtionischen Öl-in-Wasser-Emulgatoren mit einem HLB-Wert von größer 7 bis 20 ausgewählt ist.

Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seiten 913 - 916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Weitere erfindungsgemäß bevorzugte Antitranspirant-Zusammensetzungen sind dadurch gekennzeichnet, dass mindestens ein nichtionischer Emulgator mit einem HLB-Wert im Bereich von 12 - 18 enthalten ist. Bevorzugte erfindungsgemäße Antitranspirant-Zusammensetzungen sind dadurch gekennzeichnet, dass die nichtionischen ÖI-in-Wasser-Emulgatoren mit einem HLB-Wert von größer 7 bis 20 ausgewählt sind aus ethoxylierten C₈-C₂₄-Alkanolen mit durchschnittlich 10- 100 Mol Ethylenoxid pro Mol, ethoxylierten C₈-C₂₄-Carbonsäuren mit durchschnittlich 10 - 100 Mol Ethylenoxid pro Mol, mit durchschnittlich 20 - 100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, Silicon-Copolyolen mit Ethylenoxid-Einheiten oder mit Ethylenoxidund Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von größer 7 bis 20 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-10, Ceteth-12, Ceteth-20, Ceteth-30, Steareth-10, Steareth-12, Steareth-20, Steareth-21, Steareth-30, Ceteareth-10, Ceteareth-12, Ceteareth-20, Ceteareth-30, Laureth-12 und Beheneth-20.

Bevorzugte mit durchschnittlich 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80.

Weiterhin werden vorzugsweise C₈ - C₂₂-Alkylmono- und -oligoglycoside eingesetzt. C₈ -C₂₂-Alkyl-mono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} von BASF erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet. Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von größer 7 bis 20 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Besonders bevorzugte erfindungsgemäße Antitranspirant-Zusammensetzungen enthalten mindestens einen Öl-in-Wasser-Emulgator mit einem HLB-Wert von größer 7 bis 20 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung. Weitere erfindungsgemäß besonders bevorzugte Antitranspirant-Zusammensetzungen enthalten mindestens einen nichtionischen Öl-in-Wasser-Emulgator mit einem HLB-Wert im Bereich von 12 - 18 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß besonders bevorzugte Antitranspirant-Zusammensetzungen enthalten mindestens einen nichtionischen Öl-in-Wasser-Emulgator mit einem HLB-Wert im Bereich von 12 - 18, der ausgewählt ist aus linearen gesättigten und ungesättigten C₁₂ - C₂₄-Alkanolen, die mit 7 - 40 Ethylenoxid-Einheiten pro Molekül verethert sind, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung. Besonders bevorzugt sind die vorgenannten Öl-in-Wasser-Emulgatoren ausgewählt aus Steareth, Ceteth, Myristeth, Laureth, Trideceth, Arachideth und Beheneth mit jeweils 7 - 40 Ethylenoxid-Einheiten pro Molekül, insbesondere Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30, sowie Mischungen hiervon.

Weitere erfindungsgemäß bevorzugte Antitranspirant-Zusammensetzungen enthalten mindestens einen nichtionischen Öl-in-Wasser-Emulgator mit einem HLB-Wert im Bereich von 12 - 18, der ausgewählt ist aus Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30 sowie Mischungen hiervon, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß bevorzugte Antitranspirant-Zusammensetzungen enthalten mindestens ein kosmetisches Öl und mindestens einen Öl-in-Wasser-Emulgator mit einem HLB-Wert von größer 7 bis 20 enthält und liegen als Öl-in-Wasser-Emulsion vor. Im Sinne der vorliegenden Anmeldung umfasst der Begriff Emulsion keine Mikroemulsionen.

Besonders bevorzugte erfindungsgemäße Antitranspirant-Zusammensetzungen liegen als ÖI-in-Wasser-Emulsion vor und enthalten mindestens einen Öl-in-Wasser-Emulgator mit einem HLB-Wert von größer 7 bis 20 in einer Gesamtmenge von 0,5 - 5 Gew.-%, bevorzugt 0,8 - 4 Gew.-%, besonders bevorzugt 1,2-3 Gew.-%, und außerordentlich bevorzugt 1,5 - 2 Gew.-%, jeweils bezogen auf die Gesamtzusammensetzung.

Weitere erfindungsgemäß besonders bevorzugte Antitranspirant-Zusammensetzungen liegen als Öl-in-Wasser-Emulsion vor und enthalten mindestens einen nichtionischen ÖI-in-Wasser-Emulgator mit einem HLB-Wert im Bereich von 12 - 18 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß besonders bevorzugte Antitranspirant-Zusammensetzungen liegen als Öl-in-Wasser-Emulsion vor und enthalten mindestens einen nichtionischen ÖI-in-Wasser-Emulgator mit einem HLB-Wert im Bereich von 12 - 18, der ausgewählt ist aus linearen gesättigten und ungesättigten C₁₂ - C₂₄-Alkanolen, die mit 7 - 40 Ethylenoxid-Einheiten pro Molekül verethert sind, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung. Besonders bevorzugt sind die vorgenannten Öl-in-Wasser-Emulgatoren ausgewählt aus Steareth, Ceteth, Myristeth, Laureth, Trideceth, Arachideth und Beheneth mit jeweils 7 - 40 Ethylenoxid-Einheiten pro Molekül, insbesondere Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30, sowie Mischungen hiervon. Weitere erfindungsgemäß bevorzugte Antitranspirant-Zusammensetzungen liegen als ÖI-in-Wasser-Emulsion vor und enthalten mindestens einen nichtionischen Öl-in-Wasser-Emulgator mit einem HLB-Wert im Bereich von 12 - 18, der ausgewählt ist aus Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30 sowie Mischungen hiervon, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

### Wasser-in-Öl-Emulgatoren

Weitere erfindungsgemäß bevorzugte Antitranspirant-Zusammensetzungen enthalten mindestens einen Wasser-in-Öl-Emulgator, bevorzugt mindestens einen nichtionischen Wasser-in-ÖI-Emulgator, jeweils mit einem HLB-Wert größer 1,0 und kleiner/gleich 7,0, bevorzugt im Bereich von 3 - 6. Einige dieser Wasser-in-Öl-Emulgatoren sind beispielsweise in Kirk-Othmer, "Encyclopedia of Chemical Technology", 3. Aufl., 1979, Band 8, Seite 913, aufgelistet. Für ethoxylierte Addukte lässt sich der HLB-Wert, wie bereits erwähnt, auch berechnen.

Als Wasser-in-Öl-Emulgator bevorzugt sind:
- lineare oder verzweigte, gesättigte oder ungesättigte C₁₂ - C₃₀-Alkanole, die jeweils mit 1 - 4 Ethylenoxid-Einheiten pro Molekül verethert sind, welche außerordentlich bevorzugt sind aus Steareth, Ceteth, Myristeth, Laureth, Trideceth, Arachideth und Beheneth mit jeweils 1 - 4 Ethylenoxid-Einheiten pro Molekül, insbesondere Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon;
- lineare gesättigte Alkanole mit 12 - 30 Kohlenstoffatomen, insbesondere mit 16 - 22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind;
- Ester und insbesondere Partialester aus einem Polyol mit 2 - 6 C-Atomen und linearen gesättigten und ungesättigten Fettsäuren mit 12 - 30, insbesondere 14 - 22 C-Atomen, die hydroxyliert sein können. Solche Ester oder Partialester sind z. B. die Mono- und Diester von Glycerin oder Ethylenglycol oder die Monoester von Propylenglycol mit linearen gesättigten und ungesättigten C₁₂ - C₃₀-Carbonsäuren, die hydroxyliert sein können, insbesondere diejenigen mit Palmitin- und Stearinsäure, die Sorbitanmono-, -di- oder -triester von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, die Pentaerythritylmono-, -di-, -tri- und -tetraester und die Methylglucosemono- und -diester von linearen, gesättigten und ungesättigten C₁₂ - C₃₀-Carbonsäuren, die hydroxyliert sein können, wovon besonders bevorzugt sind die Mono-, Di-, Tri- und Tetraester von Pentaerythrit mit linearen gesättigten Fettsäuren mit 12 - 30, insbesondere 14 - 22 Kohlenstoffatomen, die hydroxyliert sein können, sowie Mischungen hiervon, als Konsistenzgeber und/oder Wasserbinder. Erfindungsgemäß besonders bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-ÖI-Emulgatoren mit einem HLB-Wert größer 1,0 und kleiner/ gleich 7,0 sind ausgewählt aus Glycerylmonostearat, Glyceryldistearat, Glycerylmonopalmitat, Glyceryldipalmitat und Mischungen hiervon;
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1 - 5 EO) sein können;
- Alkanole und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen,
- Glycerinmonoether gesättigter und/oder ungesättigter, verzweigter und/oder unverzweigter Alkohole einer Kettenlänge von 8 - 30, insbesondere 12 - 18 Kohlenstoffatomen,
- Partialester von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert größer 1,0 bis kleiner/gleich als 7 aufweisen,
- sowie Mischungen der vorgenannten Substanzen.

Besonders bevorzugt ist der mindestens eine Wasser-in-Öl-Emulgator mit einem HLB-Wert größer 1,0 und kleiner/gleich 7,0, bevorzugt im Bereich von 3 - 6, ausgewählt aus linearen oder verzweigten, gesättigten oder ungesättigten C₁₂ - C₃₀-Alkanole, die jeweils mit 1 - 4 Ethylenoxid-Einheiten pro Molekül verethert sind, welche außerordentlich bevorzugt sind aus Steareth, Ceteth, Myristeth, Laureth, Trideceth, Arachideth und Beheneth mit jeweils 1 - 4 Ethylenoxid-Einheiten pro Molekül, insbesondere Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon.

Erfindungsgemäß kann es bevorzugt sein, nur einen einzigen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-ÖI-Emulgatoren.

Erfindungsgemäß bevorzugte Zusammensetzungen sind dadurch gekennzeichnet, dass sie mindestens einen Wasser-in-Öl-Emulgator mit einem HLB-Wert größer 1,0 und kleiner/gleich 7,0, bevorzugt im Bereich von 3 - 6, enthalten.

Erfindungsgemäß besonders bevorzugte Antitranspirant-Zusammensetzungen enthalten mindestens einen Wasser-in-Öl-Emulgator mit einem HLB-Wert größer 1,0 und kleiner/gleich 7,0, bevorzugt im Bereich von 3 - 6, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Weitere erfindungsgemäß bevorzugte Antitranspirant-Zusammensetzungen enthalten mindestens einen nichtionischen Wasser-in-Öl-Emulgator mit einem HLB-Wert im Bereich von 3 - 6, ausgewählt aus Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Weitere erfindungsgemäß bevorzugte Antitranspirant-Zusammensetzungen liegen als Öl-in-Wasser-Emulsion vor und enthalten mindestens einen Wasser-in-Öl-Emulgator mit einem HLB-Wert größer 1,0 und kleiner/gleich 7,0, bevorzugt im Bereich von 3 - 6, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Weitere erfindungsgemäß bevorzugte Antitranspirant-Zusammensetzungen liegen als Öl-in-Wasser-Emulsion vor und enthalten mindestens einen nichtionischen Wasser-in-Öl-Emulgator mit einem HLB-Wert im Bereich von 3 - 6, ausgewählt aus Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Erfindungsgemäß bevorzugte Antitranspirant-Zusammensetzungen enthalten mindestens ein kosmetisches Öl, bevorzugt in einer Gesamtmenge von 0,1 - 15 Gew.-%, besonders bevorzugt 0,3 - 10 Gew.-%, außerordentlich bevorzugt 0,5 - 6 Gew.-%, jeweils bezogen auf das Gewicht der gesamten erfindungsgemäßen Antitranspirant-Zusammensetzung. Solche Zusammensetzungen liegen üblicherweise in Form einer Öl-in-Wasser-Emulsion vor.

Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 13 - 12000 Pa (0,1 - 90 mm Hg), besonders bevorzugt 15 - 3000 Pa, außerordentlich bevorzugt 30 - 500 Pa, aufweisen.

Erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an einoder mehrwertige C₃₋₂₂-Alkanole wie Butanol, Butandiol, Myristylalkohol und Stearylalkohol, z. B. PPG-13-Butylether, PPG-14-Butylether, PPG-9-Butylether, PPG-10-Butandiol, PPG-15-Stearylether sowie Mischungen hiervon.

Erfindungsgemäß besonders bevorzugte Zusammensetzungen enthalten mindestens ein kosmetisches Öl, ausgewählt aus PPG-13-Butylether, PPG-14-Butylether, PPG-9-Butylether, PPG-10-Butandiol, PPG-15-Stearylether sowie Mischungen hiervon, in einer Gesamtmenge von 0,1 - 15 Gew.-%, besonders bevorzugt 0,3 - 10 Gew.-%, außerordentlich bevorzugt 0,5 - 6 Gew.-%, bezogen auf das Gewicht der gesamten erfindungsgemäßen Antitranspirant-Zusammensetzung. Außerordentlich bevorzugte erfindungsgemäße Zusammensetzungen enthalten 0,1 - 15 Gew.-%, besonders bevorzugt 0,3 - 10 Gew.-%, außerordentlich bevorzugt 0,5 - 6 Gew.-%, PPG-15-Stearylether, jeweils bezogen auf das Gewicht der gesamten erfindungsgemäßen Antitranspirant-Zusammensetzung.

Weitere erfindungsgemäß besonders bevorzugte nichtflüchtige Nichtsiliconöle sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isononylisononanoat, 2-Ethylhexylpalmitat und 2-Ethylhexylstearat. Ebenfalls bevorzugt sind Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv^{®} TN (C₁₂-C₁₅-Alkylbenzoat), sowie Benzoesäureisostearylester, z. B. erhältlich als Finsolv^{®} SB, 2-Ethylhexylbenzoat, z. B. erhältlich als Finsolv^{®} EB, und Benzoesäure-2-octyldodecylester, z. B. erhältlich als Finsolv^{®} BOD. Ein weiteres besonders bevorzugtes Esteröl ist Triethylcitrat.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol und 2-Ethylhexylalkohol. Ebenfalls bevorzugt ist Isostearylalkohol. Weitere bevorzugte nichtflüchtige Öle sind ausgewählt aus Mischungen aus Guerbetalkoholen und Guerbetalkoholestern, z. B. 2-Hexyldecanol und 2-Hexyldecyllau rat.

Der im Folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol^{®} 318 oder Myritol^{®} 331 (BASF/ Cognis) mit unverzweigten Fettsäureresten sowie Glyceryltrüsostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in der erfindungsgemäßen Zusammensetzung aus.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethyl-hexylsuccinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, bevorzugt aus PPG-2-Myristylether und PPG-3-Myristylether.

Weitere erfindungsgemäß besonders bevorzugte nichtflüchtige Nichtsiliconöle sind ausgewählt aus den symmetrischen, unsymmetrischen oder cyclischen Estern der Kohlensäure mit C₆₋C₂₀-Alkoholen, z. B. Di-n-caprylylcarbonat (Cetiol^{®} CC) oder Di-(2-ethylhexyl)carbonat (Tegosoft DEC).

Ester der Kohlensäure mit C₁-C₅-Alkoholen, z. B. Glycerincarbonat oder Propylencarbonat, sind hingegen keine als kosmetisches Öl geeigneten Verbindungen.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Besonders bevorzugt beträgt das Gesamtgewicht an Dimerfettsäureestern 0,5 - 10 Gew.-%, bevorzugt 1 - 5 Gew.-%, jeweils bezogen auf die gesamte Zusammensetzung.

Flüchtige kosmetische Öle sind üblicherweise unter cyclischen Siliconölen mit der INCI-Bezeichnung Cyclomethicone ausgewählt. Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf.

Cyclomethicone sind im Stand der Technik als gut geeignete Öle für kosmetische Produkte, insbesondere für schweißhemmende und deodorierende Produkte, bekannt. Aufgrund ihrer Persistenz in der Umwelt kann es aber erfindungsgemäß bevorzugt sein, auf den Einsatz von Cyclomethiconen zu verzichten. In einer speziell bevorzugten Ausführungsform enthalten die erfindungsgemäßen Zusammensetzungen 0 bis weniger als 1 Gew.-% Cyclomethicone, bezogen auf das Gewicht der Zusammensetzung.

Ein bevorzugter Cyclomethicone-Ersatzstoff ist eine Mischung aus C13-C16 Isoparaffinen, C12 - C14 Isoparaffinen und C13 - C15 Alkanen, deren Viskosität bei 25°C im Bereich von 2 bis 6 mPas liegt und die einen Dampfdruck bei 20°C im Bereich von 100 bis 150 Pa aufweist. Eine solche Mischung ist z. B. unter der Bezeichnung SiClone SR-5 von der Firma Presperse Inc. erhältlich. Weitere bevorzugte flüchtige Siliconöle sind ausgewählt aus flüchtigen linearen Siliconölen, insbesondere flüchtigen linearen Siliconölen mit 2 - 10 Siloxaneinheiten, wie Hexamethyldisiloxan (L₂), Octamethyltrisiloxan (L₃), Decamethyltetrasiloxan (L₄), wie sie z. B. in den Handelsprodukten DC 2-1184, Dow Corning^{®} 200 (0,65 cSt) und Dow Corning^{®} 200 (1,5 cSt) von Dow Corning enthalten sind, und niedermolekulares Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist.

Bevorzugte erfindungsgemäße Antitranspirant-Zusammensetzungen enthalten wegen des trockeneren Hautgefühls und der schnelleren Wirkstofffreisetzung mindestens ein flüchtiges Nichtsiliconöl. Bevorzugte flüchtige Nichtsiliconöle sind ausgewählt aus C₈-C₁₆-Isoparaffinen, insbesondere aus Isononan, Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen, insbesondere solche mit einem Dampfdruck bei 20°C von 10 - 400 Pa, bevorzugt 13 - 100 Pa.

Weitere erfindungsgemäß bevorzugte Antitranspirant-Zusammensetzungen in Form von Öl-in-Wasser-Emulsionen sind dadurch gekennzeichnet, dass der mindestens eine Propylenglycolmonoester von verzweigten gesättigten C₆ - C₃₀-Alkancarbonsäuren ausgewählt ist aus Propylenglycolmonoisostearat, Propylenglycolmonoisopalmitat, Propylenglycolmonoisobehenat, Propylenglycolmonoisoarachinat, Propylenglycolmonoisomyristat, Propylenglycolmonoisocaprat, Propylenglycolmonoisocaprinat und Propylenglycolmonoisocaprylat sowie Mischungen hiervon. Weitere erfindungsgemäß bevorzugte Antitranspirant-Zusammensetzungen in Form von Öl-in-WasserEmulsionen sind dadurch gekennzeichnet, dass das mindestens eine verzweigte gesättigte C₁₀ - C₃₀-Alkanol ausgewählt ist aus Isostearylalkohol, Isocetylalkohol, Isomyristylalkohol, Isotridecylalkohol, Isoarachidylalkohol, Isobehenylalkohol, Isocaprylalkohol, Isocaprinylalkohol, Isocaprylylalkohol, sowie Mischungen hiervon.

Weitere erfindungsgemäß bevorzugte Antitranspirant-Zusammensetzungen in Form von Öl-in-Wasser-Emulsionen sind dadurch gekennzeichnet, dass mindestens ein nichtionischer Emulgator mit einem HLB-Wert im Bereich von 3 - 6 und mindestens ein nichtionischer Emulgator mit einem HLB-Wert im Bereich von 12 - 18 enthalten sind.

Weitere erfindungsgemäß bevorzugte Antitranspirant-Zusammensetzungen in Form von Öl-in-Wasser-Emulsionen sind dadurch gekennzeichnet, daß mindestens ein nichtionischer Emulgator mit einem HLB-Wert im Bereich von 3 - 6 in einer Gesamtmenge von 1,8 - 3 Gew.-% und mindestens ein nichtionischer Emulgator mit einem HLB-Wert im Bereich von 12 - 18 in einer Gesamtmenge von 1 - 2 Gew.-%, enthalten sind, wobei die Mengenangaben jeweils auf das Gesamtgewicht der erfindungsgemäßen Zusammensetzung bezogen sind.

Weitere erfindungsgemäß bevorzugte Antitranspirant-Zusammensetzungen in Form von Öl-in-Wasser-Emulsionen sind dadurch gekennzeichnet, dass als nichtionischer Emulgator mit einem HLB-Wert im Bereich von 3 - 6 Steareth-2 und gleichzeitig als nichtionischer Emulgator mit einem HLB-Wert im Bereich von 12 - 18 Steareth-21 enthalten ist.

Weitere erfindungsgemäß bevorzugte Antitranspirant-Zusammensetzungen in Form von Öl-in-Wasser-Emulsionen sind dadurch gekennzeichnet, dass Steareth-2, Steareth-21 und PPG-15-Stearylether enthalten sind.

Weitere erfindungsgemäß bevorzugte Antitranspirant-Zusammensetzungen enthalten insgesamt maximal 3 Gew.-%, bevorzugt maximal 1 Gew.-% und besonders bevorzugt 0 Gew.-%, jeweils bezogen auf das Gesamtgewicht der erfindungsgemäßen Zusammensetzung, an einwertigen C₁-C₃-Alkanolen, wie Ethanol oder Isopropanol.

Ganz besonders bevorzugte erfindungsgemäße Zusammensetzungen sind dadurch gekennzeichnet, dass sie mindestens ein kosmetisches Öl und mindestens einen Öl-in-Wasser-Emulgator mit einem HLB-Wert von mehr als 7 bis 20 enthalten und als Öl-in-Wasser-Emulsion, die keine Mikroemulsion darstellt, vorliegen.

Zusätzlich zu den vorgenannten Inhaltsstoffen können die erfindungsgemäßen Zusammensetzungen weitere Zusatz- und Hilfsstoffe enthalten, die beispielsweise ihre Haltbarkeit verbessern, wie Konservierungsmittel, z. B. Phenoxyethanol, Methylparaben oder Propylparaben, Antioxidantien, z. B. Tetradibutyl Pentaerythrityl Hydroxyhydrocinnamate, Lipochroman-6, Tocopherol, Tocopherylacetat oder Ascorbinsäure und deren Derivate, Vitamine und deren Derivate, wie Tocopherol, Tocopherylacetat, Ascorbinsäure, Panthenol oder Pantolacton, Parfüms, etherische Öle, Menthol und Mentholderivate, die eine hautkühlende Wirkung zeigen, Pflegestoffe mit Haut beruhigender Wirkung, wie Bisabolol und Allantoin, Wirkstoffe, die das Haarwachstum verzögern, z. B. Eflornithin oder Glycyrrhizin und dessen Derivate, Moisturizer und Feuchthaltemittel, wie 1,2-Propylenglycol, Glycerin, 2-Methyl-1,3-propandiol, 1,2-Butylenglycol, 1,3-Butylenglycol, 1,4-Butylenglycol, Pentylenglycole wie 1,2-Pentandiol und 1,5-Pentandiol, Hexandiole wie 1,2-Hexandiol und 1,6-Hexandiol, Hexantriole, wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von cis- und trans-1,4-Dimethylolcyclohexan, Harnstoff, N,N'-Bis-(2-Hydroxyethyl)harnstoff, Natriumpyrrolidoncarboxylat, Pflanzenextrakte, z. B. Aloe vera-Extrakt, natürliche Fette und Öle, wie Jojobaöl, Nachtkerzenöl oder Leinöl, gesättigte und ungesättigte Fettsäuren, wie Stearinsäure, Ölsäure, Linolsäure, Linolensäure oder gamma-Linolensäure, Squalan, Squalen, Deodorantwirkstoffe, wie Silbersalze, kolloidales Silber, Zeolithe, 2-Benzylheptan-1-ol, Anisalkohol, Mischungen von 2-Benzylheptan-1-ol und Phenoxyethanol, 3-(2-Ethylhexyloxy)-1,2-propandiol oder Tropolon, sowie Mischungen dieser Stoffe.

Die folgenden Ausführungsbeispiele sollen den Gegenstand der vorliegenden Erfindung verdeutlichen, ohne ihn hierauf zu beschränken.

### Beispiele:

Als nicht erfindungsgemäßes teilkristallines Polymer mit C₈-C₃₀-Alkyl-Seitenketten wurde Intelimer^{®} 13-1 (Fa. Air Products) verwendet. Dieses Polymer (Schmelzpunkt 48°C) wurde in den jeweils genannten Gewichtsverhältnissen mit Parfümöl bzw. hautkühlendem Wirkstoff vermischt, aufgeschmolzen und nachfolgend zu Partikeln mit zahlenmittlerer Partikelgröße von 10 bis 30 µm verarbeitet.

Alle Angaben sind in Gewichts-% (wt/wt).

### Partikel 1 (nicht erfindungsgemäß)

Intelimer IPA 13-1 Polymer / Parfumöl 1.0 / 0.5 (wt/wt)
Schmelzbereich 29-31 °C

### Partikel 2 (nicht erfindungsgemäß)

Intelimer IPA 13-1 Polymer / Mentyl Lactat 1.0 / 0.5 (wt/wt)
Schmelzbereich 33-35°C

### Partikel 3 (nicht erfindungsgemäß)

Intelimer IPA 13-1 Polymer / Mentyl Acetat 1.0 / 0.5 (wt/wt)
Schmelzpunkt 27°C

### Partikel 4 (erfindungsgemäß)

Performa V 343 Polymer / Parfumöl 1.0 / 0.5 (wt/wt)
Schmelzbereich 24-27°C

### Partikel 5

Performa V 343 Polymer / Menthyl Lacat 1.0 / 0.5 (wt/wt)
Schmelzbereich 29-34°C

### Partikel 6

Performa V 343 Polymer / Menthyl Acetat 1.0 / 0.25 (wt/wt)
Schmelzbereich 27-30°C

Mit den Partikeln wurden folgende erfindungsgemäße Zusammensetzungen hergestellt:

| | 1* | 2* | 3* | 4 | 5 | 6 |
|---|---|---|---|---|---|---|
| | Gew.-% | Gew.-% | Gew.-% | Gew.-% | Gew.-% | Gew.-% |
| Aluminum Chlorohydrat | 20,0 | 20,0 | 20,0 | 20,0 | 20,0 | 20,0 |
| Steareth-21 | 1,5 | 1,5 | 1,5 | 1,5 | 1,5 | 1,5 |
| Steareth-2 | 2,4 | 2,4 | 2,4 | 2,4 | 2,4 | 2,4 |
| PPG-15 Stearyl Ether | 0,5 | 0,5 | 0,5 | 0,5 | 0,5 | 0,5 |
| Aluminum Starch Octenylsuccinate | 0,1 | 0,1 | 0,1 | 0,1 | 0,1 | 0,1 |
| Propylene Glycol | 0,5 | 0,5 | 0,5 | 0,5 | 0,5 | 0,5 |
| EDTA | 0,1 | 0,1 | 0,1 | 0,1 | 0,1 | 0,1 |
| Dehydroxanthan Gum | 0,1 | 0,1 | 0,1 | 0,1 | 0,1 | 0,1 |
| Hydroxyethylcellulose | 0,1 | 0,1 | 0,1 | 0,1 | 0,1 | 0,1 |
| Komplex 1 | 0,25 | - | - | - | - | - |
| Komplex 2 | - | 0,5 | - | - | - | - |
| Komplex 3 | - | - | 0,1 | - | - | - |
| Komplex 4 | - | - | - | 0,25 | - | - |
| Komplex 5 | - | - | - | - | 0,5 | - |
| Komplex 6 | - | - | - | - | - | 0,2 |
| Wasser | Ad 100 | Ad 100 | Ad 100 | Ad 100 | Ad 100 | Ad 100 |

| | | | | | | |
|---|---|---|---|---|---|---|
| * nicht erfindungsgemäß | | | | | | |

## Patentansprüche

1. Partikel, enthaltend, jeweils bezogen auf das Partikelgewicht, 30 - 90 Gew.-% Polyalphaolefinwachs(e) und 10 - 70 Gew.-% eines oder mehrerer kosmetischer Wirkstoffe, ausgewählt aus Riechstoffen, Kühlwirkstoffen und Riechstoff-Kühlwirkstoff-Mischungen,
wobei das mindestens eine Polyalphaolefinwachs ausgewählt ist aus Polyalphaolefinwachsen mit einem Schmelzpunkt im Bereich von 40 - 48 °C, gemessen nach ASTM D 36, und
wobei das Partikel einen Schmelzpunkt im Bereich von 24 - 34 °C, gemessen nach ASTM D 36, aufweist.

2. Partikel nach Anspruch 1, **gekennzeichnet durch** einen zahlenmittleren Partikeldurchmesser im Bereich von 50 nm bis 100 µm, bevorzugt 100 nm bis 80 µm, besonders bevorzugt 500 nm bis 50 µm, außerordentlich bevorzugt 1 µm bis 30 µm.

3. Partikel nach einem der Ansprüche 1 oder 2, **dadurch gekennzeichnet, dass** der Anteil an Polyalphaolefinwachs(en) 50 - 75 Gew.-% und der Gesamtanteil an mindestens einem kosmetischen Wirkstoff, ausgewählt aus Riechstoffen, Kühlwirkstoffen und Riechstoff-Kühlwirkstoff-Mischungen, 25 - 50 Gew.-%, beträgt, jeweils bezogen auf das Partikelgewicht.

4. Partikel nach einem der Ansprüche 1 bis 3, **gekennzeichnet durch** einen Schmelzpunkt im Bereich von 27 - 32°C, außerordentlich bevorzugt 29 - 31 °C, gemessen nach ASTM D 36.

5. Kosmetische Zusammensetzung, enthaltend Wasser und 0,01 - 10 Gew.-%, bezogen auf das Gewicht der kosmetischen Zusammensetzung, eines oder mehrerer Partikel gemäß Anspruch 1, 2, 3 oder 4, wobei mindestens ein Antitranspirant-Wirkstoff enthalten ist.

6. Kosmetische Zusammensetzung gemäß Anspruch 5, **dadurch gekennzeichnet, dass** mindestens ein Deodorant-Wirkstoff enthalten ist.

7. Kosmetische Zusammensetzung gemäß Anspruch 5 oder 6, **dadurch gekennzeichnet, dass** die Zusammensetzung eine Antitranspirant-Zusammensetzung ist und mindestens einen Öl-in-Wasser-Emulgator mit einem HLB-Wert von größer 7 bis 20 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, enthält, jeweils bezogen auf die Gesamtzusammensetzung.

8. Kosmetische Zusammensetzung gemäß Anspruch 5, 6 oder 7, **dadurch gekennzeichnet, dass** die Zusammensetzung eine Antitranspirant-Zusammensetzung ist und mindestens einen nichtionischen Öl-in-Wasser-Emulgator mit einem HLB-Wert im Bereich von 12 -18 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, enthält, jeweils bezogen auf die Gesamtzusammensetzung.

9. Kosmetische Zusammensetzung gemäß Anspruch 5, 6, 7 oder 8, **dadurch gekennzeichnet, dass** die Zusammensetzung eine Antitranspirant-Zusammensetzung ist und mindestens einen nichtionischen Öl-in-Wasser-Emulgator mit einem HLB-Wert im Bereich von 12 - 18, der ausgewählt ist aus linearen gesättigten und ungesättigten C₁₂ - C₂₄-Alkanolen, die mit 7 - 40 Ethylenoxid-Einheiten pro Molekül verethert sind, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, enthält, jeweils bezogen auf die Gesamtzusammensetzung.

10. Kosmetische Zusammensetzung gemäß Anspruch 5, 6, 7, 8 oder 9, **dadurch gekennzeichnet, dass** die Zusammensetzung eine Antitranspirant-Zusammensetzung ist und mindestens einen nichtionischen Wasser-in-Öl-Emulgator mit einem HLB-Wert im Bereich von 3 - 6, ausgewählt aus Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon, in einer Gesamtmenge von 1,8 - 3 Gew.-%, bevorzugt 2 - 2,8 Gew.-% und besonders bevorzugt 2,4 - 2,6 Gew.-%, enthält, jeweils bezogen auf das Gesamtgewicht der erfindungsgemäßen Zusammensetzung.

11. Kosmetische Zusammensetzung gemäß Anspruch 5, 6, 7, 8, 9 oder 10, **dadurch gekennzeichnet, dass** mindestens ein nichtionisches verdickendes Polymer enthalten ist, das ausgewählt ist aus Cellulose und Celluloseethern sowie Mischungen hiervon, bevorzugt ausgewählt aus Hydroxyethylcellulose, in einer Gesamtmenge von 0,05 -1 Gew.-%, bevorzugt 0,1 - 0,7 Gew.-%, besonders bevorzugt 0,1 - 0,3 Gew.-%, jeweils bezogen auf das Gesamtgewicht der Zusammensetzung.

12. Kosmetische Zusammensetzung gemäß Anspruch 5, 6, 7, 8, 9, 10 oder 11, **dadurch gekennzeichnet, dass** Dehydroxanthan Gum in einer Menge von 0,05 - 1 Gew.-%, besonders bevorzugt 0,1 - 0,8 Gew.-%, außerordentlich bevorzugt 0,2 - 0,5 Gew.-%, jeweils bezogen auf das Gesamtgewicht der Zusammensetzung, enthalten ist.

13. Kosmetische Zusammensetzung gemäß Anspruch 5, 6, 7, 8, 9, 10, 11 oder 12, **dadurch gekennzeichnet, dass**, jeweils bezogen auf das Gesamtgewicht der Zusammensetzung, 0,05 - 1,0 Gew.-%, bevorzugt 0,1 - 0,8 Gew.-%, besonders bevorzugt 0,2 - 0,5 Gew.-%, Dehydroxanthan Gum und 0,05 -1 Gew.-%, bevorzugt 0,1 - 0,7 Gew.-%, besonders bevorzugt 0,1 - 0,3 Gew.-%, Hydroxyethylcellulose, enthalten ist.

## Claims

1. A particle which contains, in each case based on the particle weight, 30 to 90 wt.% polyalphaolefin wax(es) and 10 to 70 wt.% of one or more cosmetic active ingredients, selected from odorants, cooling active ingredients and mixtures of odorants and cooling active ingredients,
wherein the at least one polyalphaolefin wax is selected from polyalphaolefin waxes having a melting point in the range of from 40 to 48°C, measured in accordance with ASTM D 36, and
wherein the particle has a melting point in the range of from 24 to 34°C, measured in accordance with ASTM D 36.

2. The particle according to claim 1, **characterized by** a number average particle diameter in the range of from 50 nm to 100 µm, preferably from 100 nm to 80 µm, particularly preferably from 500 nm to 50 µm and extremely preferably from 1 µm to 30 µm.

3. The particle according to claim 1 or 2, **characterized in that** the proportion of polyalphaolefin wax(es) is 50 to 75 wt.% and the total proportion of at least one cosmetic active ingredient, selected from odorants, cooling active ingredients and mixtures of odorants and cooling active ingredients, is 25 to 50 wt.%, in each case based on the particle weight.

4. The particle according to one of claims 1 to 3, **characterized by** a melting point in the range of from 27 to 32°C, extremely preferably from 29 to 31°C, measured in accordance with ASTM D 36.

5. A cosmetic composition which contains water and, based on the weight of the cosmetic composition, 0.01 to 10 wt.% of one or more particles according to claims 1, 2, 3 or 4, wherein at least one antiperspirant active ingredient is contained.

6. The cosmetic composition according to claim 5, **characterized in that** at least one deodorant active ingredient is contained.

7. The cosmetic composition according to claim 5 or 6, **characterized in that** the composition is an antiperspirant composition and contains at least one oil-in-water emulsifier having an HLB value of from greater than 7 to 20 in a total amount of from 0.5 to 5 wt.%, preferably from 0.8 to 4 wt.%, particularly preferably from 1.2 to 3 wt.% and extremely preferably from 1.5 to 2 wt.%, in each case based on the total composition.

8. The cosmetic composition according to claim 5, 6 or 7, **characterized in that** the composition is an antiperspirant composition and contains at least one non-ionic oil-in-water emulsifier having an HLB value in the range of from 12 to 18 in a total amount of from 0.5 to 5 wt.%, preferably from 0.8 to 4 wt.%, particularly preferably from 1.2 to 3 wt.% and extremely preferably from 1.5 to 2 wt.%, in each case based on the total composition.

9. The cosmetic composition according to claim 5, 6, 7 or 8, **characterized in that** the composition is an antiperspirant composition and contains at least one non-ionic oil-in-water emulsifier having an HLB value in the range of from 12 to 18 which is selected from linear saturated and unsaturated C₁₂ to C₂₄ alkanols etherified with 7 to 40 ethylene oxide units per molecule, in a total amount of from 0.5 to 5 wt.%, preferably from 0.8 to 4 wt.%, particularly preferably from 1.2 to 3 wt.% and extremely preferably from 1.5 to 2 wt.%, in each case based on the total composition.

10. The cosmetic composition according to claim 5, 6, 7, 8 or 9, **characterized in that** the composition is an antiperspirant composition and contains at least one non-ionic water-in-oil emulsifier having an HLB value in the range of from 3 to 6 which is selected from steareth-2, steareth-3, steareth-4, ceteth-2, ceteth-3, ceteth-4, myristeth-2, myristeth-3, myristeth-4, laureth-2, laureth-3, laureth-4, trideceth-2, trideceth-3 and trideceth-4 and mixtures thereof, in a total amount of from 1.8 to 3 wt.%, preferably from 2 to 2.8 wt.% and particularly preferably from 2.4 to 2.6 wt.%, in each case based on the total weight of the composition according to the invention.

11. The cosmetic composition according to claim 5, 6, 7, 8, 9 or 10, **characterized in that** at least one non-ionic thickening polymer is contained, selected from cellulose and cellulose ethers and mixtures thereof, preferably selected from hydroxyethyl cellulose, in a total amount of from 0.05 to 1 wt.%, preferably from 0.1 to 0.7 wt.% and particularly preferably from 0.1 to 0.3 wt.%, in each case based on the total weight of the composition.

12. The cosmetic composition according to claim 5, 6, 7, 8, 9, 10 or 11, **characterized in that** dehydroxanthan gum is contained in an amount of from 0.05 to 1 wt.%, particularly preferably from 0.1 to 0.8 wt.% and extremely preferably from 0.2 to 0.5 wt.%, in each case based on the total weight of the composition.

13. The cosmetic composition according to claim 5, 6, 7, 8, 9, 10, 11 or 12, **characterized in that**, in each case based on the total weight of the composition, 0.05 to 1.0 wt.%, preferably 0.1 to 0.8 wt.% and particularly preferably 0.2 to 0.5 wt.% dehydroxanthan gum and 0.05 to 1 wt.%, preferably 0.1 to 0.7 wt.% and particularly preferably 0.1 to 0.3 wt.% hydroxyethyl cellulose is contained.

## Revendications

1. Particule contenant, respectivement rapporté au poids de la particule, 30 - 90 % en poids de cire de poly-alpha-oléfine et 10 - 70 % en poids d'un ou plusieurs principe(s) actif(s) cosmétique(s), sélectionné(s) parmi les substances odorantes, les agents refroidissants et les composés de substances odorantes et d'agents refroidissants,
dans laquelle l'au moins une cire de poly-alpha-oléfine est sélectionnée parmi les cires de poly-alpha-oléfine dont le point de fusion est compris dans une plage allant de 40 à 48 °C mesuré selon ASTM D 36, et dans laquelle la particule présente un point de fusion compris dans une plage allant de 24 à 34 °C mesuré selon ASTM D 36.

2. Particule selon la revendication 1, **caractérisée en ce qu'**elle possède un diamètre de particule moyen compris dans une plage allant de 50 nm à 100 µm, préférablement de 100 nm à 80 µm, très préférablement de 500 nm à 50 µm, on ne peut plus préférablement de 1 µm à 30 µm.

3. Particule selon l'une quelconque des revendications 1 ou 2, **caractérisée en ce que** la part en cire(s) de poly-alpha-oléfine s'élève entre 50 et 75 % en poids et la part totale en au moins un principe actif cosmétique, sélectionné parmi les substances odorantes, les agents refroidissants et les composés de substances odorantes et d'agents refroidissants, s'élève entre 25 et 50 % en poids, respectivement rapporté au poids de la particule.

4. Particule selon l'une quelconque des revendications 1 à 3, **caractérisée en ce qu'**elle possède un point de fusion compris dans une plage de 27 à 32 °C, on ne peut plus préférablement de 29 à 31 °C mesuré selon ASTM D 36.

5. Composition cosmétique contenant de l'eau et de 0,01 à 10 % en poids, rapporté au poids de la composition cosmétique, d'une ou de plusieurs particules selon la revendication 1, 2, 3 ou 4, laquelle contenant au moins un principe actif antitranspirant.

6. Composition cosmétique selon la revendication 5, **caractérisée en ce qu'**elle contient au moins un principe actif désodorisant.

7. Composition cosmétique selon la revendication 5 ou 6, **caractérisée en ce que** la composition est une composition antitranspirante et contient au moins un émulsifiant huile-dans-l'eau dont la valeur HLB (équilibre hydrophile/lipophile) de plus de 7 à 20 dans une quantité totale de 0,5 à 5 % en poids, préférablement de 0,8 à 4 % en poids, très préférablement de 1,2 à 3 % en poids et on ne peut plus préférablement de 1,5 à 2 % en poids, respectivement rapporté à la composition totale.

8. Composition cosmétique selon la revendication 5, 6 ou 7, **caractérisée en ce que** la composition est une composition antitranspirante et contient au moins un émulsifiant huile-dans-l'eau non-ionique doté d'une valeur HLB dans une plage allant de 12 à 18 dans une quantité totale de 0,5 à 5 % en poids, préférablement de 0,8 à 4 % en poids, très préférablement de 1,2 à 3 % en poids et on ne peut plus préférablement de 1,5 à 2 % en poids, respectivement rapporté à la composition totale.

9. Composition cosmétique selon la revendication 5, 6, 7 ou 8, **caractérisée en ce que** la composition est une composition antitranspirante et contient au moins un émulsifiant huile-dans-l'eau non-ionique avec une valeur HLB comprise dans la plage de 12 à 18, qui est sélectionné parmi les alcanols C₁₂-C₂₄ linéaires saturés et non-saturés, qui sont étherifiés avec 7 - 40 unités d'oxyde d'éthylène par molécule, dans une quantité totale de 0,5 à 5 % en poids, préférablement de 0,8 à 4 % en poids, très préférablement de 1,2 à 3 % en poids et on ne peut plus préférablement de 1,5 à 2 % en poids, respectivement rapporté à la composition totale.

10. Composition cosmétique selon la revendication 5, 6, 7, 8 ou 9, **caractérisée en ce que** la composition est une composition antitranspirante et contient au moins un émulsifiant eau-dans-l'huile non-ionique avec une valeur HLB comprise dans une plage allant de 3 à 6, sélectionné parmi le stéareth-2, le stéareth-3, le stéareth-4, le ceteth-2, le ceteth-3, le ceteth-4, le mysriteth-2, le myristeth-3, le myristeth-4, le laureth-2, le laureth-3, le laureth-4, le trideceth-2, le trideceth-3 et le trideceth-4, ainsi que des composés de ceux-ci, dans une quantité totale de 1,8 à 3 % en poids, préférablement de 2 à 2,8 % en poids et très préférablement de 2,4 à 2,6 % en poids, respectivement rapporté au poids total de la composition selon l'invention.

11. Composition cosmétique selon la revendication 5, 6, 7, 8, 9 ou 10, **caractérisée en ce qu'**elle contient au moins un polymère épaississant non-ionique, qui est sélectionné parmi la cellulose et les éthers de cellulose ainsi que des composés de ceux-ci, préférablement sélectionnés parmi l'hydroxyéthylcellulose, dans une quantité totale de 0,05 à 1 % en poids, préférablement de 0,1 à 0,7 % en poids, très préférablement de 0,1 à 0,3 % en poids, respectivement rapporté au poids total de la composition.

12. Composition cosmétique selon la revendication 5, 6, 7, 8, 9, 10 ou 11, **caractérisée en ce qu'**elle contient de la gomme de déhydroxanthane dans une quantité de 0,05 à 1 % en poids, très préférablement de 0,1 à 0,8 % en poids, on ne peut plus préférablement de 0,2 à 0,5 % en poids, respectivement rapporté au poids total de la composition.

13. Composition cosmétique selon la revendication 5, 6, 7, 8, 9, 10, 11 ou 12, **caractérisée en ce que**, respectivement rapporté au poids total de la composition, elle contient de 0,05 à 1,0 % en poids, préférablement 0,1 à 0,8 % en poids, très préférablement de 0,2 à 0,5 % en poids en gomme déhydroxanthane et de 0,05 à 1 % en poids, préférablement de 0,1 à 0,7 % en poids, très préférablement de 0,1 à 0,3 % en poids en hydroxyéthylcellulose.
